# Europäisches Patentamt

# European Patent Office

# Office européen des brevets

(19)

(11) Veröffentlichungsnummer: **0 152 648**
**B1**

(12)

# EUROPÄISCHE PATENTSCHRIFT

(45) Veröffentlichungstag der Patentschrift:
**12.08.87**

(21) Anmeldenummer: **84201907.7**

(22) Anmeldetag: **19.12.84**

(51) Int. Cl.⁴: **C 07 C 29/15,** C 07 C 31/04, C 07 C 31/02

(54) **Verfahren zur Herstellung einer Mischung aus Methanol und Höheren Alkoholen.**

(30) Priorität: **02.02.84 DE 3403492**

(43) Veröffentlichungstag der Anmeldung:
**28.08.85 Patentblatt 85/35**

(45) Bekanntmachung des Hinweises auf die Patenterteilung:
**12.08.87 Patentblatt 87/33**

(84) Benannte Vertragsstaaten:
**DE FR GB IT NL SE**

(56) Entgegenhaltungen:
AT-B-281 777
AU-B-6 171 380
DE-A-3 203 748
DE-A-3 310 540
DE-B-2 846 614
FR-A-2 114 792
FR-A-2 181 002
FR-A-2 471 963

THE CANADIAN JOURNAL OF CHEMICAL
ENGINEERING, Band 61, Februar 1983 K.J. SMITH et
al. "The Higher Alcohol Synthesis over Promoted
Cu/ZnO Catalysts" Seiten 40-45

(73) Patentinhaber: **METALLGESELLSCHAFT AG,
Reuterweg 14 Postfach 3724, D-6000 Frankfurt/M.1
(DE)**

(72) Erfinder: **Cornelius, Gerhard, Dornbachweg 2,
D-6367 Karben 4 (DE)**
Erfinder: **Hilsebein, Wolfgang, Am Eisernen
Schlag 7, D-6000 Frankfurt 50 (DE)**
Erfinder: **König, Peter, Dr., Am Alten Schloss 79,
D-6000 Frankfurt 50 (DE)**
Erfinder: **Möller, Friedrich, Dr., Breslauer Ring 15,
D-6382 Friedrichsdorf (DE)**
Erfinder: **Supp, Emil, Marburger Strasse 7, D-6057
Dietzenbach (DE)**

(74) Vertreter: **Rieger, Harald, Dr., Reuterweg 14,
D-6000 Frankfurt am Main (DE)**

**0 152 648**

**Beschreibung**

Die Erfindung betrifft ein Verfahren zur Herstellung einer Mischung aus Methanol und Höheren Alkoholen mit 2 bis 7 C-Atomen pro Molekül aus einem Wasserstoff und Kohlenoxide enthaltenden Synthesegas an einem Kupfer, Zink und Aluminium enthaltenden Katalysator bei einer Temperatur im Bereich von 200 bis 320°C und einem Druck im Bereich von 50 bis 150 bar.

Aus der DE-OS 33 10 540 ist ein solches Verfahren bekannt, wobei der zu verwendende Katalysator als wesentliche Elemente Kupfer, Kobalt, Aluminium sowie mindestens ein Alkali- oder Erdalkalimetall enthält. Auch Zink kann im bekannten Katalysator vorliegen.

Smith und Anderson berichten im Canadian Journal of Chemical Engineering, Band 61 (Februar 1983), Seiten 40 bis 45, über Untersuchungen zur Synthese Höherer Alkohole an einem alkalihaltigen $CuO-ZnO-Al_2O_3$-Katalysator. Aus dem über den Katalysator geleiteten Synthesegas bilden sich Methanol und Höhere Alkohole, wobei der Gehalt an Höheren Alkoholen im Flüssigprodukt deutlich abhängig ist vom Gehalt des $K_2CO_3$-Aktivators im Katalysator. Die höchste Ausbeute an Höheren Alkoholen wurde mit einem Gehalt von 0,5 Gew.% $K_2CO_3$ im Katalysator festgestellt, bei niedrigeren Gehalten an $K_2CO_3$ fiel die Ausbeute an Höheren Alkoholen rasch ab.

Der Erfindung liegt die Aufgabe zugrunde, das eingangs genannte Verfahren mit hohen Ausbeuten sowohl an Methanol als auch an Höheren Alkoholen durchzuführen und lange Standzeiten des Katalysators zu erreichen. Derartige Methanol-Alkoholgemische finden in der Technik Anwendung als Oktanzahlverbesserer und Lösungsvermittler für Wasser im Gemisch mit natürlichen Otto-Kraftstoffen. Gleichzeitig soll der Wassergehalt im Produkt der Synthese niedrig gehalten werden, um eine destillative Aufarbeitung zu vermeiden, die wegen der entstehenden Azeotropbildung schwierig ist. Die Begrenzung des Wassergehaltes ist deshalb außerordentlich wichtig. Die Lösung der Aufgabe besteht beim eingangs genannten Verfahren darin, daß man einen praktisch erdalkalifreien Katalysator-Vorläufer bestehend aus 35 bis 65 Gew.% CuO, 15 bis 45 Gew.% ZnO, 5 bis 20 Gew.% $Al_2O_3$ und höchstens 0,1 Gew.% Kalium verwendet, dessen Oxide vor Beginn der Synthese mindestens teilweise reduziert werden, und daß man dem Katalysator ein Synthesegas mit einen Mol-Verhältnis $H_2 : CO$ von 0,3 bis 1,9 und mit einem $CO_2$-Gehalt von höchstens 2 Vol.% zuführt. Der Katalysator ist frei von Kobalt.

Überraschenderweise wurde gefunden, daß man immer dann ein sehr wasserarmes Produkt erhält, wenn der $CO_2$-Gehalt in dem zum Katalysator gelangenden Synthesegas niedrig ist und das $H_2/CO$-Molverhältnis deutlich den Zahlwert 2 unterschreitet. Der den Katalysator verlassende Gasstrom enthält kondensierbare Flüssigprodukte, wobei folgende Komponenten üblicherweise das Flüssigprodukt bilden:

| | |
|---|---|
| Methanol: | 55 - 88 Gew.% |
| Höhere Alkohole: | 10 - 45 Gew.% |
| ($C_2$ - $C_7$) | |
| Wasser: | 0,1 - 1,5 Gew.% |

Beim erfindungsgemäßen Verfahren, das vorzugsweise im Temperaturbereich von 250 bis 300°C durchgeführt wird, kommt dem Katalysator naturgemäß hohe Bedeutung zu. Es ist zweckmäßig daß der Katalysator-Vorläufer sowie der für die Synthese mindestens teilweise reduzierte Katalysator ein Gewichtsverhältnis Cu : Zn im Bereich von 0,7 bis 4,8 und vorzugsweise von 1,0 bis 2,4 aufweist.

Aufgrund bekannt gewordener Untersuchungen, wie etwa die von Smith und Anderson, war nicht vorherzusehen, daß ein weitgehend alkalifreier Cu-Zn-Al-Katalysator auch hervorragende Ergebnisse bei der gleichzeitigen Synthese von Methanol und Höheren Alkoholen erbringen würde. Aktivierende Zusätze von Alkali waren ja bisher stets als für dieses Ziel notwendig dargestellt worden.

Man kann die Produktion an Höheren Alkoholen noch weiter dadurch steigern, daß das dem Katalysator zugeführte Synthesegas Methanoldampf, z. B. in einem Volumenanteil von 3 bis 15 %, enthält.

Die bevorzugten Katalysatoren erhält man z. B. dadurch, daß die katalytisch wirksamen Kupferoxid-Zinkoxid-Komponenten aus wäßrigen Lösungen der entsprechenden Salze (z. B. Nitrate oder Acetate) mit alkalisch reagierenden Substanzen in Gegenwart des kolloidal (als Gel oder Sol) verteilten Aluminiumoxids oder Aluminiumhydroxids ausgefällt werden. Das erhaltene Gemisch bzw. Fällungsprodukt kann in an sich bekannter Weise getrocknet, kalziniert, zu Formkörpern verpreßt und gegebenenfalls reduziert werden. Vorzugsweise verwendet man als alkalisch reagierende Substanzen für die Fällung ein alkali- oder Ammoniumkarbonat oder -bikarbonat, vorzugsweise Kaliumkarbonat oder Kaliumbikarbonat. Es hat sich gezeigt, daß zum Erzeugen auch Höherer Alkohole im Syntheseprodukt ein gewisser niedriger Kaliumgehalt nicht stört und erheblich günstiger als ein entsprechender Natriumgehalt ist.

Zur Verbesserung der Porenstruktur im Katalysator empfiehlt es sich, die Fällung der Kupferoxid-Zinkoxid-Komponente in Gegenwart des kolloidal verteilten Aluminiumoxids oder -hydroxids unter Verwendung von verdünnten Lösungen der alkalisch reagierenden Substanzen, z. B. 10 - 20 Gew.% Alkalicarbonat, bei niedrigen Temperaturen und bei neutralen oder schwach sauren pH-Werten durchzuführen. Die Fällungstemperatur beträgt zweckmäßigerweise 25 bis 65°C, vorzugsweise 30 bis 40°C. Der Zusatz der alkalisch reagierenden Substanzen wird im allgemeinen beendet, wenn der pH-Wert im Bereich von 6,5 bis 7,5 liegt.

Für eine gesteigerte Ausbeute an Höheren Alkoholen empfiehlt sich ein Katalysator, der im oxidischen Zustand ein Porenvolunen von 0,25 bis 0,5 $cm^3/g$ besitzt. Besonders wichtig ist auch die Porenverteilung dieses

2

Katalysator-Vorläufers, wobei 50 bis 85 % des Porenvolumens zu Poren mit einem Durchmesser im Bereich von 0,014 bis 0,08 μm, 15 bis 50 % des Porenvolumens zu Poren mit einem Durchmesser kleiner 0,014 μm und höchstens 4 % des Porenvolumens zu Poren mit einem Durchmesser größer als 0,08 μm gehören sollen. Dieses Porenvolumen wird nach der Methode der Quecksilberporosimetrie bestimmt (Literatur: R. Anderson, Experimental Methods in Catalytic Research, Academic Press, New York, 1968).

Für den Fachmann ist ersichtlich, daß der zuvor erläuterte Katalysatortyp auch für die Methanolsynthese mit nur sehr geringen Mengen an Höheren Alkoholen verwendbar ist, was durch Versuche nachgewiesen wurde (siehe Beispiel 1). Hierbei wird ein $H_2$-reiches Synthesegas verwendet.

**Beispiele für die Katalysatorherstellung:**

**Katalysator A**

Zur Fällung des Katalysatorvorläufers werden zwei Lösungen hergestellt:

Lösung 1: 418 g Kupfernitrat und 50 g Zinkoxid werden in 1,6 l Wasser und 148 g 52 %iger Salpetersäure gelöst und anschließend mit kolloidalem Aluminiummetahydrat-Gel versetzt.

Lösung 2: 535 g Kaliumkarbonat werden in 3317 g Wasser gelöst. Die Lösungen werden bei 40°C unter starkem Rühren in der Weise vereinigt, daß der pH-Wert während der Fällung 6,9 beträgt. Nach Abschluß der Fällung, wobei der pH-Wert 7,1 noch nicht überschritten hat, wird der Niederschlag abfiltriert und mit Wasser gewaschen, bis im ablaufenden Filtrat kein Alkali (d.h. Kalium) mehr nachzuweisen ist. Der Filterkuchen wird bei 120°C getrocknet und anschließend 8 Stunden bei 280°C kalziniert. Das kalzinierte Produkt zerkleinert man und verpreßt es nach Zusatz von 2 Gew.% Graphit.

Der geglühte, graphitfreie Katalysatorvorläufer besteht zu 65 Gew.% aus CuO, zu 23 Gew.% aus ZnO und zu 12 Gew.% aus $Al_2O_3$. Der Kaliumgehalt in dem so hergestellten Katalysatorvorläufer A beträgt 0,06 Gew.%. Die physikalischen Eigenschaften der hergestellten Tabletten mit der Dimension 5 x 5 mm sind folgende:

Tabletten-Schüttgewicht: 1025 g/l
Porenvolumen: 0,36 cm$^3$/g

Porenverteilung, durch Hg-Porosimetrie bestimmt:

Porendurchmesser 0,08 bis 0,014 μm: 78 % des Porenvolumens
Porendurchmesser kleiner 0,014 μm: 21 % des Porenvolumens
Porendurchmesser über 0,08 μm: 1 % des Porenvolumens

**Katalysator B**

Der Katalysatorvorläufer B wird in analoger Weise wie Katalysator A hergestellt, wobei als einzige Abänderung der abfiltrierte Filterkuchen nur zweimal mit Wasser gewaschen wird. Dadurch ist das ablaufende Filtrat nicht alkalifrei.

Der geglühte, graphitfreie Katalysatorvorläufer besteht zu 65 Gew.% aus CuO, zu 23 Gew.% aus ZnO und zu 12 Gew.% aus $Al_2O_3$, der Restgehalt an Alkali beträgt 0,31 Gew.% Kalium. In den physikalischen Eigenschaften des Katalysators B sind keine Unterschiede zum Katalysator A festzustellen.

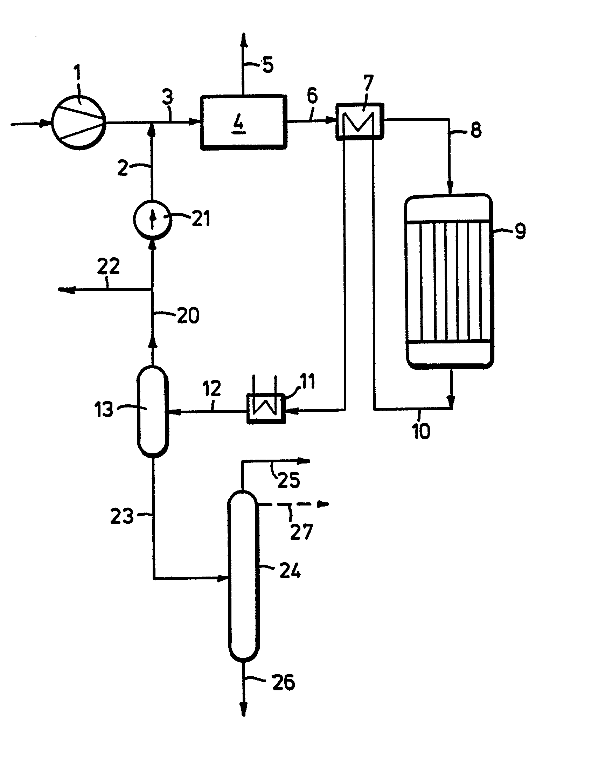

Eine Verfahrensführung wird mit Hilfe der Zeichnung erläutert.

Wasserstoff und Kohlenoxide enthaltendes Synthesegas wird durch den Kompressor 1 herangeführt und mit Restgas aus der Leitung 2 gemischt. Das Gemisch wird in der Leitung 3 einer Verfahrensstufe 4 zur Entfernung von $CO_2$ zugeführt, wo auf an sich bekannte Weise das $CO_2$ weitgehend aus dem Gasgemisch entfernt wird. Das dabei freiwerdende $CO_2$-Gas in der Leitung 5 kann vorteilhaft für die Erzeugung des Synthesegases Verwendung finden, wenn dieses z. B. aus Naturgas hergestellt wird. Das Auswaschen von $CO_2$ kann z. B. mittels Methanol oder einem Gemisch aus Methanol und Höheren Alkoholen erfolgen, wobei sich Produkt der Verfahrensführung verwenden läßt.

Das von $CO_2$ weitgehend befreite Gas gelangt durch die Leitung 6 in einen Wärmeaustauscher 7, wo es erwärmt wird und strömt dann durch die Leitung 8 zum Synthesereaktor 9. Der Reaktor 9 ist als Röhrenreaktor ausgebildet, dessen Röhren den vor allem aus Kupfer, Zink und Aluminium bestehenden Katalysator enthalten. Zwischen den Röhren befindet sich ein flüssiges Kühlmittel, z. B. Wasser.

Das in den Reaktor 9 durch die Leitung 8 eintretende Synthesegas besteht vor allem aus $H_2$ und CO, deren Molverhältnis im Bereich von 0,3 bis 1,9 liegt. Der Gehalt an CO beträgt höchstens 2 Vol.%. Im Katalysator des Reaktors liegen die Temperaturen im Bereich von 200 bis 320°C und vorzugsweise bei etwa 250 bis 300°C der Druck beträgt 50 bis 150 bar. Im Reaktor findet die Umsetzung des Synthesegases zu Methanol und Höheren Alkoholen statt.

Das Produkt der Synthese verläßt den Reaktor 9 durch die Leitung 10 und wird zunächst im Wärmeaustauscher 7 mit dem kalten Gas aus der Leitung 6 indirekt gekühlt, zur weiteren Kühlung folgt ein Wasserkühler 11. Das teilweise kondensierte Produkt wird in der Leitung 12 zum Abscheider 13 geführt, wo die Restgase abgetrennt und durch die Leitung 20 abgezogen werden. Ein Teil dieser Restgase wird über den Kreislaufverdichter 21 und die Leitung 2 zurückgeführt. Ein Zweigstrom der Restgase wird über die Leitung 22 abgezogen.

Das im Abscheider 13 anfallende flüssige Produkt führt man in der Leitung 23 zu einer Destillation 24, wo man die Leichtsieder, vor allem Dimethyläther, Methylformiat und Aceton abtrennt und über Kopf durch die Leitung 25 ableitet. Das Sumpfprodukt der Destillationskolonne 24 besteht aus Methanol und Höheren Alkoholen mit einem Wassergehalt von höchstens etwa 2 Gew.%; es wird in der Leitung 26 abgeführt und eignet sich ganz besonders als Motortreibstoff, insbesondere auch als Zusatz zu Normal- oder Superbenzin für Otto-Motoren.

Falls erwünscht, kann die Konzentration der Höheren Alkohole in dem aus Leitung 26 abgeführten Produkt dadurch erhöht werden, daß auch noch Methanol in der Destillation 24 abdestilliert und entweder über Kopf zusammen mit den Leichtsiedern durch Leitung 25 oder aus einem Seitenabzug 27 separat aus der Kolonne 24 abgeführt wird.

Man kann die Produktion an Höheren Alkoholen auch dadurch steigern, daß das dem Katalysator des Reaktors zugeführte Synthesegas Methanoldampf, z. B. in einem Volumenanteil von 3 bis 15 %, enthält. Diesen Methanoldampf-Anteil kann man dadurch erzeugen, daß man das Methanol im Produkt der Leitungen 10 und 12 nur unvollständig kondensiert, so daß das Restgas in der Leitung 2 Methanoldampf enthält. Ferner kann man auch Methanol in das Synthesegas der Leitung 8 einspritzen.

Der Restgas-Teilstrom der Leitung 20 kann ganz oder teilweise für eine zweite, nicht dargestellte Methanolsynthese verwendet werden. Diese zweite Methanolsynthese kann in bekannter Weise so betrieben werden, daß ihr Produkt vor allem aus Methanol besteht und daneben nur geringe Mengen an Höheren Alkoholen aufweist. Eine solche Methanolsynthese kann einen vor allem aus Kupfer, Zink und Aluminium bestehenden Katalysator aufweisen, wie er im Reaktor 9 eingesetzt wird. Da das Restgas der Leitung 20 erhebliche Mengen an CO und auch $CO_2$ aufweist, muß dieses Restgas vor Eintritt in die zweite Methanolsynthese mit Wasserstoff angereichert werden, damit das $H_2/CO$-Verhältnis mindestens 2,0 beträgt.

Die mit Hilfe der Zeichnung erläuterte Verfahrensführung ist auch dazu geeignet, um als Produkt des Synthesereaktors 9 vor allem Methanol ohne nennenswerte Mengen an Höheren Alkoholen zu erzeugen, wenn ein Synthese-Frischgas mit einem $H_2/CO$-Verhältnis größer 2 eingesetzt wird. Dieses Synthese-Frischgas wird vom Kompressor 1 angesaugt, es führt zu einem $H_2$-reichen, über den Katalysator geleiteten Synthesegas.

**Beispiel 1**

100 cm³ des Katalysators A werden in einen Rohrreaktor, der mit einer Wassermantelkühlung versehen ist, eingebaut und drucklos mit einer Mischung aus 1 Vol.% $H_2$ und 99 % $N_2$ reduziert, wobei die Temperatur im Reaktor stufenweise auf 240°C angehoben wird. Bei 240°C wird Synthesegas eingeleitet, welches aus 44 Vol.% $CO_2$, 10 Vol.% CO, 75 Vol.% $H_2$ und 11 Vol.% Inerten besteht. Unter den gewählten Versuchsbedingungen, Druck 50 bar, Belastung pro Liter Katalysator 10 400 Nl/h, erhält man nach etwa 100 - 150 Betriebsstunden eine Flüssigausbeute von 1,105 kg/h pro Liter Katalysator mit folgender Zusammensetzung:

| | |
|---|---|
| Methanol | 94,25 Gew.% |
| Äthanol | 0,08 Gew.% |
| Propanole | 0,04 Gew.% |
| Butanole | 0,03 Gew.% |
| $H_2O$ | 5,6 Gew.% |

Nach 150 Betriebsstunden wird die Temperatur auf 270°C und der Druck auf 100 bar erhöht. Bei einer Synthesegasbelastung pro Liter Katalysator von 4 500 Nl/h und einer Zusammensetzung des Synthesegases aus 0,5 Vol.% $CO_2$, 48 Vol.% CO, 50 Vol.% $H_2$ und 1,5 Vol.% Inerten wird nach der 150. Betriebsstunde eine flüssige Produktausbeute pro Liter Katalysator von 1,02 kg/h mit folgender Zusammensetzung erhalten:

| | |
|---|---|
| Methanol | 84,7 Gew.% |
| Äthanol | 6,0 Gew.% |
| Propanole | 3,2 Gew.% |
| Butanole | 3,5 Gew.% |
| Pentanole und andere Höhere Alkohole | 2,3 Gew.% |
| Wasser | 0,3 Gew.% |

Aus diesem Beispiel sieht man, daß man an einem nahezu alkalifreien Katalysator (0,06 Gew.% Kalium) sowohl eine Mischung aus Methanol und Höheren Alkoholen (z. B. 15 Gew.%) als auch ein an Höheren Alkoholen (0,15 Gew.%) armes Methanol erzeugen kann.

4

**Beispiel 2**

100 cm³ des Katalysators B mit einem Alkaligehalt von 0,31 Gew.% Kalium werden in den Reaktor des Beispiels 1 eingesetzt und wie dort beschrieben reduziert.

Bei 270°C und 100 bar werden pro Liter Katalysator mit 4500 Nl/h Synthesegas, bestehend aus 0,5 Vol.% $CO_2$, 48 Vol.% CO, 50 Vol.% $H_2$ und 1,5 Vol.% Inerten, im Reaktor umgesetzt. Zwischen der 150. und 200. Betriebsstunde erhält man pro Liter Katalysator eine Ausbeute an flüssigem Produkt von 0,98 kg/h, das folgende Zusammensetzung hat:

| | |
|---|---|
| Methanol | 90,9 Gew.% |
| Äthanol | 3,5 Gew.% |
| Propanole | 1,8 Gew.% |
| Butanole | 1,9 Gew.% |
| Hexanole und andere Höhere Alkohole | 1,7 Gew.% |
| Wasser | 0,2 Gew.% |

Aus diesem Versuch folgt, daß der Katalysator B mit einem Gehalt von 0,31 Gew.% Kalium unter gleichen Bedingungen nahezu die gleiche Ausbeute an flüssigen Produkten erbringt wie der Katalysator A, der bis an die Grenze des in der Praxis sinnvollen von Alkali (Kalium-Restgehalt 0,06 Gew.%) befreit ist.

Der entscheidende Unterschied aber ist, daß der alkaliarme Katalysator A ein Produkt mit weit höherem Gehalt an $C_2$-$C_7$-Alkoholen (15,0 Gew.%) erzeugt als der alkalireiche Katalysator B mit 8,9 Gew.% $C_2$- und Höheren Alkoholen im flüssigen Produkt.

**Beispiel 3**

Der Katalysator A wird in den Reaktor des Beispiels 1 eingesetzt und nach der Reduktion zuerst mit einem $CO_2$-armen Synthesegas (Gas I) und dann mit einem $CO_2$-reichen Synthesegas (Gas II) beaufschlagt. Die Versuchsbedingungen und die dabei erzielten Versuchsergebnisse sind nachstehend aufgeführt:

# 0 152 648

| | Gas I | Gas II |
|---|---|---|
| **Synthesegas** | | |
| $CO_2$ Vol.% | 1,4 | 11,4 |
| CO Vol.% | 61,0 | 60,1 |
| $H_2$ Vol.% | 30,0 | 26,1 |
| Inerte Vol.% | 7,6 | 2,4 |
| | | |
| Druck (bar) | 50 | 50 |
| Temperatur (°C) | 275 | 275 |
| Belastung (Nl/l h) | 2 650 | 2 700 |
| | | |
| **Flüssigprodukt-Ausbeute** | | |
| (kg/l h) | 0,21 | 0,16 |
| | | |
| **Zusammensetzung des Flüssig-Produktes** | | |
| Methanol Gew.% | 80,0 | 86,4 |
| Höhere Alkohole Gew.% | 19,8 | 12,1 |
| Wasser Gew.% | 0,2 | 1,5 |

Aus dieser Tabelle geht hervor, daß bei erhöhtem $CO_2$-Gehalt im dem Katalysator zugeführten Synthesegas die Erzeugung Höherer Alkohole deutlich abnimmt und der Wassergehalt im Flüssigprodukt ansteigt, wodurch u.U. die Toleranzgrenze für den Wassergehalt im Kraftstoffgemisch überschritten wird.

**Patentansprüche**

1. Verfahren zur Herstellung einer Mischung aus Methanol und Höheren Alkoholen mit 2 bis 7 C-Atomen pro Molekül aus einem Wasserstoff und Kohlenoxide enthaltenden Synthegas an einem Kupfer, Zink und Aluminium enthalenden Katalysator bei einer Temperatur im Bereich von 200 bis 320°C und einem Druck im Bereich von 50 bis 150 bar, dadurch gekennzeichnet, daß man einen praktisch erdaikalifreien Katalysator-Vorläufer bestehend aus 35 bis 65 Gew.% CuO, 15 bis 45 Gew.% ZnO, 5 bis 20 Gew.% $Al_2O_3$ und höchstens 0,1 Gew.% Kalium werwendet, dessen Oxide vor Beginn der Synthese mindestens teilweise reduziert werden, und daß man dem Katalysator ein Synthesegas mit einem Mol-Verhältnis $H_2$ : CO on 0,3 bis 1,9 und mit einem $CO_2$-Gehalt von höchstens 2 Vol.% zuführt.

2. Verfahren nach Anspruch 1, dadurch gekennzeichnet, daß man die Synthese bei Temperaturen im Bereich von 250 bis 300 °C durchführt.

3. Verfahren nach Anspruch 1 oder 2, dadurch gekennzeichnet, daß der Katalysator-Vorläufer sowie der für die Synthese mindestens teilweise reduzierte Katalysator ein Gewichtsverhältnis von Cu : Zn im Bereich von 0,7 bis 4,8 und vorzugsweise von 1,0 bis 2,4 aufweist.

4. Verfahren nach Anspruch 1 oder einem der folgenden, dadurch gekennzeichnet, daß der Katalysator-Vorläufer ein Porenvolumen von 0,25 bis 0,5 $cm^3$/g aufweist.

5. Verfahren nach Anspruch 1 oder einem der folgenden, dadurch gekennzeichnet, daß im Katalysator-Vorläufer 50 bis 85 % des Porenvolumens zu Poren mit einem Durchmesser im Bereich von 0,014 bis 0,08 µm, 15

6

bis 50 % des Porenvolumens zu Poren mit einem Durchmesser kleiner 0,014 μm und höchstens 4 % des Porenvolumens zu Poren mit einem Durchmesser größer als 0,08 μm gehören.

6. Verfahren nach Anspruch 1 oder einem der folgenden, dadurch gekennzeichnet, daß in dem kondensierbaren Anteil des den Katalysator verlassenden Produktes nicht mehr als 2 Gew.% Wasser enthalten sind.

7. Verfahren nach Anspruch 1 oder einem der folgenden, dadurch gekennzeichnet, daß man aus dem Synthesegas vor der Synthese $CO_2$ mit Hilfe von im Reaktionsprodukt enthaltenem Methanol oder einem Methanol-Alkoholgemisch auswäscht.

8. Verfahren nach Anspruch 1 oder einem der folgenden, dadurch gekennzeichnet, daß das dem Katalysator zugeführte Synthesegas zu 3 bis 15 Vol.% aus Methanoldampf besteht.

## Claims

1. Process of producing a mixture of methanol and higher alcohols having 2 to 7 carbon atoms per molecule from a synthesis gas containing hydrogen and carbon oxides by a reaction carried out at a temperature in the range from 200 to 320° C on a copper, zinc and aluminum containing catalyst and under a pressure in the range from 50 to 100 bars, characterized in that a catalyst precursor is used, which contains 35 to 65 wt.% CuO, 15 to 45 wt.% ZnO, 5 to 20 wt.% $Al_2O_3$ and up to 0.1 wt.% potassium, the oxides of said catalyst are reduced at least in part before the synthesis begins, and the catalyst is contacted with a synthesis gas having a $H_2$ : CO mole ratio of 0.3 to 1.9 and a $CO_2$ content not in excess of 2 vol.%.

2. Process according to claim 1, characterized in that the synthesis is carried out at temperatures in the range from 250 to 300° C.

3. Process according to claim 1 or 2, characterized in that in the catalyst precursor and in the catalyst which has been at least partly reduced for the synthesis the weight ratio of Cu:Zn is in the range from 0.7 to 4.8 and preferably in the range from 1.0 to 2.4.

4. Process according to claim 1 or any of the following claims, characterized in that the catalyst precursor has a void volume of 0.25 to 0.5 cm³/g.

5. Process according to claim 1 or any of the following claims, characterized in that in the catalyst precursor 50 to 85 % of the void volume are constituted by pores which are 0.014 to 0.08 μm in diameter, 15 to 50 % by pores which are less than 0.014 μm in diameter, and not in excess of 4 % of the void volume by pores which are in excess of 0.08 μm in diameter.

6. Process according to claim 1 or any of the following claims, characterized in that the condensible fraction of the product withdrawn from the catalyst contains water not in excess of 2 wt.%.

7. Process according to claim 1 or any of the following claims, characterized in that $CO_2$ is removed from the synthesis gas by means of methanol contained in the reaction product or by means of a methanol-alcohol mixture before the synthesis gas is used for the synthesis.

8. Process according to claim 1 or any of the preceding claims, characterized in that the synthesis gas contacted with the catalyst contains 3 to 15 vol.% methanol vapor.

## Revendications

1. Procédé de préparation d'un mélange de méthanol et d'alcools supérieurs ayant de 2 à 7 atomes de carbone par molécule, à partir d'un gaz de synthèse contenant de l'hydrogène et des oxydes de carbone, sur un catalyseur contenant du cuivre, du zinc et de l'aluminium à une température comprise entre 200 et 320° C et sous une pression comprise entre 50 et 150 bar, caractérisé en ce qu'il consiste à utiliser un précurseur de catalyseur pratiquement exempt de métal alcalino-terreux constitué de 35 à 65 % en poids de CuO, de 15 à 45 % en poids de ZnO, de 5 à 20 % en poids d'$Al_2O_3$ et au plus de 0,1 % en poids de potassium et dont les oxydes sont réduits au moins partiellement avant le début de la synthèse, et à amener au catalyseur un gaz de synthèse ayant un rapport molaire $H_2$ : CO de 0,3 à 1,9 et une teneur en $CO_2$ de 2 % en volume au plus.

2. Procédé suivant la revendication 1, caractérisé en ce qu'il consiste à effectuer la synthèse à des températures comprises entre 250 et 300° C.

3. Procédé suivant la revendication 1 ou 2, caractérisé en ce que le précurseur de catalyseur ainsi que le catalyseur au moins partiellement réduit pour la synthèse, ont un rapport pondéral de Cu : Zn compris entre 0,7 et 4,8 et, de préférence, compris entre 1,0 et 2,4.

4. Procédé suivant la revendication 1 ou l'une des suivantes, caractérisé en ce que le précurseur de catalyseur a un volume de pores de 0,25 à 0,5 cm³/g.

5. Procédé suivant la revendication 1 ou l'une des suivantes, caractérisé en ce que, dans le précurseur de catalyseur, de 50 à 85 % du volume des pores appartiennent à des pores ayant un diamètre compris entre 0,014 et 0,08 micron, de 15 à 50 % du volume des pores appartiennent à des pores ayant un diamètre inférieur à 0,014 micron et au plus 4 % du volume des pores appartiennent à des pores ayant un diamètre supérieur à 0,08 micron.

6. Procédé suivant la revendication 1 ou l'une des suivantes, caractérisé en ce que, dans la partie condensable du produit quittant le catalyseur, il n'y a pas plus de 2 % en poids d'eau.

7. Procédé suivant la revendication 1 ou l'une des suivantes, caractérisé en ce qu'il consiste à éliminer $CO_2$ avant la synthèse par lavage du gaz de synthèse à l'aide de méthanol contenu dans le produit de réaction ou d'un mélange d'alcool et de méthanol.

8. Procédé suivant la revendication 1 ou l'une des suivantes, caractérisé en ce que le gaz de synthèse amené au catalyseur est constitué, pour 3 à 15 % en volume, de vapeur de méthanol.